Europäisches Patentamt

European Patent Office

Office européen des brevets

(19)

(11) Numéro de publication: **0 315 496 B1**

## (12) FASCICULE DE BREVET EUROPEEN

(45) Date de publication de fascicule du brevet: **22.09.93**

(51) Int. Cl.5: **C12P 19/18**, C12P 7/58, //C12R1:685

(21) Numéro de dépôt: **88401804.5**

(22) Date de dépôt: **11.07.88**

(54) **Procédé de préparation conjointe d'oligosides riches en fructose et d'acide gluconique par voie fermentaire.**

(30) Priorité: **29.10.87 FR 8715015**

(43) Date de publication de la demande:
**10.05.89 Bulletin 89/19**

(45) Mention de la délivrance du brevet:
**22.09.93 Bulletin 93/38**

(84) Etats contractants désignés:
**AT BE CH DE ES FR GB GR IT LI LU NL SE**

(56) Documents cités:

PATENT ABSTRACTS OF JAPAN, vol. 11, no. 132 (C-417)[2579], 24 avril 1987

PATENT ABSTRACTS OF JAPAN, vol. 11, no. 47 (C-403)[2494], 13 février 1987

CHEMICAL ABSTRACTS, vol. 100, 1984, pages 445-446, résumé no. 84456x, Columbus, Ohio, US

(73) Titulaire: **SOCIETE GENERALE POUR LES TECHNIOUES NOUVELLES S.G.N. Société anonyme dite:**
**1, rue des Hérons Montigny-le-Bretonneux**
**F-78184 Saint-Ouentin-en-Yvelines Cédex(FR)**

(72) Inventeur: **Goma, Gérard**
**20 rue du Salas**
**F-31520 Ramonville-St-Agne(FR)**
Inventeur: **Seiller, Isabelle**
**17 avenue des Iles**
**F-31650 St-Orens-de-Gameville(FR)**
Inventeur: **Bajon, Anne-Marie**
**64 rue de Grégovie**
**F-75014 Paris(FR)**
Inventeur: **Leygue, Jean-Philippe**
**Rés."Jardins Occitants" 95 av. Tolosane,Bât.12**
**31520 Ramonville-St-Agne(FR)**

(74) Mandataire: **Le Roux, Martine et al**
**Cabinet Beau de Loménie 158, rue de l'Université**
**F-75340 Paris Cédex 07 (FR)**

Rank Xerox (UK) Business Services
(3.10/3.6/3.3.1)

EP 0 315 496 B1

CHEMICAL ABSTRACTS, vol. 85, 1976, page 673, résumé no. 124254k, Columbus, Ohio, US; M. TOMODA et al.: "Production of several oligosaccharides from sucrose by the action of an Aspergillus enzyme preparation and structural studies of the products"

**Description**

L'invention se situe dans le domaine de la demande de brevet FR-85/15027 déposée par la demanderesse.

Ladite demande de brevet décrit un procédé de préparation de fructose et d'acide gluconique (ou gluconate) par voie fermentaire à partir de solutions de saccharose. Un seul micro-organisme est utilisé du type Aspergillus et notamment Aspergillus niger. Une seule étape de procédé est nécessaire. Les solutions contiennent moins de 200 g/l en saccharose.

La demanderesse a constaté qu'en modifiant certaines conditions dudit procédé, des oligosides riches en fructose peuvent être préparés.

Ces derniers sont d'un intérêt certain pour l'alimentation. Ils ont un pouvoir sucrant mais ne présentent pas les inconvénients du saccharose : effet cariogène et inversion immédiate en glucose et fructose avec absorption rapide de glucose sans dépendance de l'insuline.

Ainsi, on a proposé de remplacer le saccharose par un mélange glucose-fructose plus riche en fructose par exemple à 70 % de fructose.

On a vu apparaître des oligosides riches en fructose contenant de 2 à 9 molécules de fructose pour une molécule de glucose, ce que l'on écrit GFn avec n = 2, 3,...9.

La demande de brevet français FR-81/06308 décrit un procédé d'obtention par voie exclusivement enzymatique, d'oligosaccharides à partir de saccharose sous l'action d'une préparation enzymatique de fructosyl-transférase soit d'origine microbienne, soit d'origine végétale.

Le document propose comme source d'enzymes divers micro-organismes, en particulier des champignons du genre Aspergillus (Aspergillus niger,...) du genre Penicillium, du genre Fusarium, des levures du genre Saccharomyces, genre Pichia et des espèces végétales comme Asparagus officinalis, Hélianthus tuberosus...

L'inconvénient de ce procédé est de séparer la sécrétion de l'enzyme et son action catalytique sur le sucre. En outre, le procédé ne parle pas de production d'acide gluconique.

La demande de brevet français FR-86/10106 décrit un procédé d'obtention d'oligosaccharides à partir de saccharose par voie exclusivement enzymatique avec élimination du glucose par oxydation enzymatique, soit microbienne.

Le procédé selon la présente invention offre la particularité de faire agir directement en une seule étape sur la solution de saccharose un seul micro-organisme qui présente les potentialités de fabriquer, à partir de solutions concentrées en saccharose, des oligosides riches en fructose, du fructose pur et d'éliminer le glucose par oxydation en acide gluconique, ce avec de bons rendements.

Ledit procédé consiste à placer des cellules proliférantes d'Aspergillus niger au contact de solutions concentrées à plus de 400 g/l, en saccharose et à un pH voisin de 5, avec une alimentation régulée en solution de saccharose et en un liquide basique qui transforme l'acide gluconique en gluconate.

Conditions opératoires

La fermentation est effectuée en aérobiose par des cellules de préférence en phase proliférante (nombre de cellules en accroissement), à la rigueur en fin de phase proliférante.

Les souches utilisées sont de préférence des souches d'Aspergillus niger.

Dans tous les cas, le milieu de culture a la composition suivante :
- saccharose à concentration variable supérieure à 400 g/l, suivant les modes de fermentation, et de préférence supérieure à 750 g/l. Pour démarrer les réactions de fermentation, les solutions sont diluées, par exemple, à environ 160 g/l, puis la concentration est augmentée graduellement jusqu'à la valeur choisie pour le fonctionnement.
- sels minéraux nécessaires à la croissance fongique :

| | |
|---|---|
| . $Ca(NO_3)_2, 4H_2O$ | environ 1 g/l |
| . $NH_4H_2PO_4$ | environ 1 g/l |
| . KCl | environ 0,25 g/l |
| . $MgSO_4, 7H_2O$ | environ 0,25 g/l |
| . $FeCl_3$ | environ 0,01 g/l |

La température de culture est comprise entre 20 et 40°C, de préférence égale à 30°C.

3

L'agitation est calculée de manière optimale en fonction des conditions opératoires requises. Dans la plupart des expériences, l'agitation est de 800 tr/min.

L'aération est assurée par de l'air stérile à raison d'au moins 1 volume d'air par volume réactionnel et par minute (noté par la suite 1 vvm).

Le pH peut être régulé ou non. Le pH peut être régulé à une valeur comprise entre 3,0 et 7,0 et de préférence 5,0 par addition d'une base (solution de soude, de potasse, lait de chaux, chaux en poudre, carbonate, autre source azotée,...).

Le procédé de préparation comprend une première étape, dans laquelle les souches Aspergillus niger sont cultivées en aérobiose dans une solution de saccharose diluée, de préférence à moins de 200 g/l en saccharose, 150 g/l exemple, ladite solution étant additionnée des nutriments nécessaires et agitée.

L'évolution de la réaction est suivie par dosage de glucose ou par dosage des triosides $GF_2$, ou suivie de tout paramètre y étant corrélé.

Lorsque la concentration en glucose avoisine 10 g/l ou lorsque la concentration en triosides est proche de son maximum, la concentration en saccharose est augmentée jusqu'à sa valeur nominale en fonctionnement, valeur supérieure à 400 g/l et de préférence supérieure à 750 g/l.

En effet, il a été constaté qu'il fallait maintenir dans le milieu une concentration résiduelle en glucose afin d'assurer une vitesse optimale de production d'acide gluconique. Une concentration à 10 g/l en glucose a été choisie pour optimiser la production simultanée en fructose et acide gluconique, ce qui correspond à une production en acide gluconique de 10,6 g/l.h.

La réaction d'oxydation de glucose étant de loin la principale consommatrice d'oxygène, on peut relier la diminution de cette vitesse de production d'acide gluconique à une augmentation de la concentration en oxygène dissous. La mesure de ce paramètre permet donc de suivre également la réaction.

Il a été observé que la polymérisation en GFn commence par la production de $GF_2$ puis après un maximum de $GF_2$ les oligosides $GF_3$, $GF_4$... se forment. Ainsi, le maximum de la courbe des triosides correspond à un optimum dans l'activité des microorganismes. Ce critère a été retenu pour déterminer le moment auquel la concentration en saccharose peut être élevée.

En fonctionnement, l'alimentation avec la solution concentrée en saccharose peut être faite à débit constant, elle peut également être asservie à la régulation du pH ou à la concentration en oxygène dissous.

On peut opérer en mode discontinu, semi-continu ou continu, ces 2 derniers modes étant préférés.

En effet, les essais menés par la demanderesse ont montré que l'hydrolyse des oligosides, réaction à cinétique lente qui est favorisée par un abaissement du pH, ne commence que lorsque le saccharose est épuisé. Cela correspond en mode continu ou semi-continu à l'arrêt de l'alimentation et en mode discontinu à l'épuisement de la charge initiale en saccharose.

L'avancement de la réaction est suivi par mesure du pH et de la pression partielle en oxygène dissous.

Les produits sont dosés au cours de la réaction, par chromatographie liquide haute performance par exemple.

Dans le procédé ci-décrit, les enzymes synthétisées pendant la croissance fongique ont pour effet :

- de transférer n groupements fructosyl à partir de n molécules de saccharose conduisant à (n-1) molécules de glucose G et une molécule d'hétéro-oligosides de structure GFn,

$$nGF \longrightarrow (n-1) \; G \; + \; GFn$$

- d'hydrolyser les oligosides formés en glucose, fructose et oligosides de degré de polymérisation inférieure,
- et dans tous les cas d'oxyder le glucose libéré en acide gluconique.

Pour simplifier l'écriture, on appelle F le fructose, G le glucose, AG l'acide gluconique, FG le saccharose, $GF_2$, $GF_3$, $GF_4$,... les oligosides et Fn les oligofructosides.

On parlera en résumé d'oligosides pour désigner sans distinction les GFn et les Fn.

La présence des molécules de glucose est le plus souvent dévalorisante par suite de leur faible valeur et de la difficulté à les extraire du milieu réactionnel.

Le mélange ainsi formé d'acide gluconique et d'oligosides conduit à l'obtention d'une part de l'acide gluconique ou d'un gluconate et, d'autre part, des oligosides riches en fructose ou du fructose par des opérations de purification simple et sélective (résines échangeuses d'ions, précipitation au méthanol, électrodialyse). Les oligosides conduisent par hydrolyse à des solutions très riches en fructose.

Pour éclairer la description, les exemples suivants présentent :

- une fermentation semi-continue avec alimentation en substrat contrôlée par la mesure de la pression partielle en oxygène dissous,

- une fermentation semi-continue avec alimentation à débit constant,
- deux fermentations semi-continues avec alimentation dépendante de la régulation de pH.

Exemple 1 : Fermentation semi-continue (alimentation régulée)

La fermentation avec alimentation programmée en substrat est effectuée dans un réacteur infiniment mélangé BIOLAFITTE de 20 l, équipé d'un système de régulation de pH et d'un boîtier d'asservissement de l'alimentation à la concentration en oxygène dissous.

La souche utilisée est A. niger INSG 492.

L'agitation est de 800 tr/min.

L'aération est de 1 vvm (augmentée par incréments couplés au débit d'alimentation).

Le pH est régulé à 5 avec une solution de soude 10N. L'inoculum représente le 1/10ème du volume initial. La fermentation se déroule comme suit :
- pied de cuve initial de 7 l avec une solution initiale de saccharose à 165 g/l complétée en sels minéraux,
- après 15 h de culture soit quand la concentration de glucose dans le milieu est voisine de 10 g/l, le réacteur est alimenté avec une solution de saccharose concentrée à 873 g/l. Cette alimentation est asservie à une concentration en oxygène dissous de 1,5 mg/l correspondant à la vitesse maximale d'oxydation du glucose.

A 15 h de culture, les concentrations sont les suivantes :

| | |
|---|---|
| . acide gluconique | 53 g/l |
| . fructose | 16 g/l |
| . saccharose | 12 g/l |
| . glucose | 8 g/l |
| . $GF_2 + F_3$ | 52 g/l |
| . $GF_3 + F_4$ | 42 g/l |
| . $GF_4 + F_5$ | 6 g/l |

A 45 h de culture, le volume de fermenteur est de 15 l, le milieu de culture a la composition suivante :

```
. acide gluconique.............. 223  g/l

. fructose...................... 16  g/l

. saccharose.................... 30  g/l

. glucose......................  5  g/l
```

$$
\left.
\begin{array}{ll}
. GF_2 + F_3 ........................ & 41 \ \ g/l \\
. GF_3 + F_4 ........................ & 56 \ \ g/l \\
. GF_4 + F_5 ........................ & 69 \ \ g/l \\
. GF_5 + F_6 ........................ & 46 \ \ g/l \\
. GF_6 + F_7 \ \text{à}\ GF_8 + F_9 ........... & <0,05 \ g/l
\end{array}
\right\} = 212 \ g/l
$$

Le rendement carbone est de 98%. La productivité est de 5 g/l.h en acide gluconique.

Après 63 h de culture, l'alimentation en saccharose concentré est arrêtée. Le volume réactionnel est alors de 18,5 l.

Les résultats des dosages sont les suivants :

```
. acide gluconique............... 250 g/l
. fructose...................... 24 g/l
. saccharose.................... 14 g/l
. glucose...................... 2 g/l   ⎫
. GF₂ + F₃.................... 34 g/l  ⎪
. GF₃ + F₄.................... 56 g/l  ⎬ = 206 g/l
. GF₄ + F₅.................... 60 g/l  ⎪
. GF₅ + F₆.................... 56 g/l  ⎭
```

Le rendement carbone est de 90 %.

Le rendement en acide gluconique est égal à 80 % du rendement théorique. Cependant, la concentration en glucose sous toutes ses formes (oxydée en acide gluconique ou dans le motif des oligosides) est de 59 g/l, ce qui ramène le rendement molaire à sa valeur théorique. Le déficit du rendement peut être attribué à une consommation du fructose.

Exemple 2 : fermentation semi-continue avec alimentation à débit constant

Les conditions de culture sont données ci-dessous :
- réacteur CHEMAP de volume 20 l,
- agitation : 800 tr/min,
- aération : 1,1 vvm (débit augmenté graduellement avec le volume réactionnel),
- pH régulé à 5 avec une solution de soude 10N,
- Inoculum : Aspergillus niger INSG 492 : 1/10ème du volume initial.
  La culture s'est déroulée en 2 temps :
- culture discontinue de 6 l avec une concentration initiale en saccharose de 150 g/l complémentée en sels minéraux,
- alimentation à débit constant égal à 0,162 l/h avec un sirop de saccharose à 873 g/l.
  L'alimentation est commencée quand la teneur en glucose atteint 10 g/l dans le pied de cuve.
  Le volume final est de 18,5 l.
  A l'arrêt de la culture (t = 83,5 h), la composition du milieu est la suivante :

```
. acide gluconique............. 322    g/l
. fructose.................... 157    g/l
. saccharose................. 18    g/l
. glucose.................... 2    g/l
. GF₂ + F₃.................... 31    g/l  ⎫
. GF₃ + F₄.................... 19    g/l  ⎪
. GF₄ + F₅.................... 18    g/l  ⎬ = 108 g/l
. GF₅ + F₆.................... 40    g/l  ⎪
. GF₆ + F₇ à GF₈ + F₉.......... <0,05 g/l  ⎭
```

Les oligosides, non compris le saccharose résiduel, représentent environ 38 % des sucres totaux.

Exemple 3 : fermentation semi-continue avec alimentation dépendante de la régulation de pH.

Les conditions de culture sont identiques à celles de l'exemple 2.
La culture s'est déroulée comme suit :
- culture discontinue de 5 l avec une concentration initiale en saccharose de 180 g/l complémentée en sels minéraux,
- commencement de l'alimentation (solution de saccharose à 873 g/l) quand la concentration en triosides $GF_2$ est maximale.

Le milieu de culture a alors la composition suivante :

| . acide gluconique | 15 g/l |
|---|---|
| . fructose | 20 g/l |
| . saccharose | 49,5 g/l |
| . glucose | 32 g/l |
| . $GF_2 + F_3$ | 69 g/l |
| . $GF_3 + F_4$ | 11 g/l |

Les oligosides (hors saccharose) représentent environ 45 % des sucres restants.
L'alimentation est couplée au volume du liquide régulateur de pH dans un rapport 9,5.
A l'arrêt de l'alimentation (t = 50 h), le volume final est de 17 l, les concentrations sont les suivantes :

. acide gluconique............... 138 g/l

. fructose...................... 58 g/l

. saccharose.................... 108 g/l

. glucose....................... 48 g/l

. $GF_2 + F_3$..................... 199 g/l ⎫

. $GF_3 + F_4$..................... 91 g/l ⎬

. $GF_4 + F_5$..................... 16 g/l ⎬ = 328 g/l

. $GF_5 + F_6$..................... 2,2 g/l ⎬

. $GF_6 + F_7$..................... <0,05 g/l ⎭

Les oligosides (hors saccharose) représentent 59 % des sucres totaux.
Le rendement en acide gluconique par rapport au saccharose est de 0,28 g/g, soit 49 % du rendement théorique.
Le rendement (par rapport au saccharose) en fructose est de 0,12 g/g.
Le rendement en $GF_2 + F_3$ est de 0,41 g/g
Le rendement en $GF_3 + F_4$ est de 0,19 g/g
Le rendement en $GF_4 + F_5$ est de 0,03 g/g
La productivité en acide gluconique est de 2,76 g/l.h.
La productivité totale en oligosides est de 6,18 g/l.h.

A 80 h (soit 30 h après l'arrêt de la culture), la composition du milieu est la suivante :

```
.  acide gluconique...........  259    g/l
.  fructose....................   55    g/l
.  saccharose..................   18    g/l
.  glucose.....................  0,4    g/l
.  GF2 + F3....................   97    g/l  ⎫
.  GF3 + F4....................  124    g/l  ⎪
.  GF4 + F5....................   54    g/l  ⎬  = 281 g/l
.  GF5 + F6....................    6    g/l  ⎪
.  GF6 + F7.................... <0,05  g/l  ⎭
```

Le oligosides (hors saccharose) représentent environ 80 % des sucres totaux.
Le rendement carbone est de 103 %.
La productivité en acide gluconique est alors de 3,25 g/l.h.
La productivité globale en oligosides est de 3,54 g/l.h.

Exemple 4 : fermentation semi-continue avec alimentation dépendante de la régulation de pH dès le début de la culture dans un rapport 6,1.

Les conditions de culture sont identiques à celles de l'exemple 3.
Le milieu de culture à 50 h (fin de l'alimentation) à la composition suivante :

```
.  acide gluconique...........  140  g/l
.  fructose....................   24  g/l
.  saccharose..................   24  g/l
.  glucose.....................   31  g/l
.  GF2 + F3....................  100  g/l  ⎫
.  GF3 + F4....................  143  g/l  ⎪
.  GF4 + F5....................   46  g/l  ⎬  = 292 g/l
.  GF5 + F6....................    3  g/l  ⎭
```

Les oligosides (hors saccharose) représentent environ 79 % des sucres totaux.
Le rendement en acide gluconique par rapport au saccharose est de 0,31 g/g.
Le rendement en fructose (par rapport au saccharose) est de 0,05 g/g.
Le rendement en $GF_2$ + $F_3$ (par rapport au saccharose) est de 0,23 g/g.
Le rendement en $GF_3$ + $F_4$ (par rapport au saccharose) est de 0,32 g/g.
Le rendement en $GF_4$ + $F_5$ (par rapport au saccharose) est de 0,10 g/g.
La productivité en acide gluconique est de 2,8 g/l.h.
La productivité totale en oligosides est de 5,84 g/l.h.
A 80 h, soit 30 h après l'arrêt de l'alimentation en substrat, la composition du milieu est la suivante :

| . acide gluconique | 165 g/l |
|---|---|
| . fructose | 28 g/l |
| . saccharose | 17 g/l |
| . glucose | 39 g/l |
| . $GF_2 + F_3$ | 65 g/l |
| . $GF_3 + F_4$ | 121 g/l |
| . $GF_4 + F_5$ | 85 g/l |
| . $GF_5 + F_6$ | 12 g/l |

Les oligosides (hors saccharose) représentent environ 77 % des sucres totaux.

Le rendement en acide gluconique est de 0,34 g/g.

La productivité en acide gluconique est de 2,06 g/l.h.

La productivité totale en oligosides est de 3,53 g/l.h.

Le rendement total de conversion reste très voisin de 100 %.

Entre 50 et 80 h, le rendement en acide gluconique + glucose passe de 0,67 à 0,75 mole/mole de saccharose.

Par contre, la productivité en oligosides chute fortement de 5,84 à 3,53 g/l.h.

La productivité en oligosides est maximale après 24,5 h de culture et est égale à 8,7 g/l.h. (mais le saccharose résiduel est alors de 45 g/l).

Les cinq exemples précédents montrent que le procédé de la demanderesse permet de fabriquer conjointement, dans un seul réacteur, sand séparation des phases de réaction, à partir de saccharose, l'acide gluconique et les oligosides au moins jusqu'à $GF_5$.

Le saccharose mis en oeuvre est pratiquement entièrement consommé et on trouve très peu de glucose comme sous-produit.

## Revendications

1. Procédé de préparation aérobie conjointe d'acide gluconique, de fructose et d'oligosides dans lequel on fait agir sur des solutions de saccharose des micro-organismes Aspergillus niger en maintenant la solution à un pH voisin de 5 par addition régulée d'un liquide basique transformant l'acide gluconique en gluconate, caractérisé en ce que :
   - un pied de cuve est préparé par fermentation d'Aspergillus niger dans la solution de saccharose diluée,
   - lorsque la concentration en glucose est d'environ 10 g/l ou lorsque la concentration en triosides, $GF_2$ est proche de son maximum, la concentration en saccharose est augmentée à plus de 400 g/l sur des cellules proliférantes d'Aspergillus niger,
   - les micro-organismes sont séparés du milieu de culture avant épuisement de la concentration en saccharose pour éviter l'hydrolyse des oligosides,
   - lesdits oligosides et fructose sont séparés de l'acide gluconique ou du gluconate.

2. Procédé selon la revendication 1, caractérisé en ce que la concentration en saccharose est d'au moins 750 g/l.

3. Procédé selon la revendication 1, caractérisé en ce que l'on opère en fonctionnement continu ou semi-continu.

## Claims

1. Process for the simultaneous aerobic preparation of gluconic acid, fructose and oligosaccharides in which Aspergillus niger microorganisms are allowed to act on sucrose solutions while the solution is maintained at a pH in the vicinity of 5 by the controlled addition of a basic liquid which converts gluconic acid to gluconate, characterised in that:
   - a yeast sediment is prepared by Aspergillus niger fermentation in the diluted sucrose solution,
   - when the glucose concentration is about 10 g/l, or when the concentration of trisaccharides $GF_2$ is close to its maximum, the sucrose concentration is increased to above 400 g/l over proliferating Aspergillus niger cells,

9

- the microorganisms are separated from the culture medium before depletion of the sucrose concentration so as to avoid hydrolysis of the oligosaccharides,
- the said oligosaccharides and fructose are separated from the gluconic acid or the gluconate.

2. Process according to Claim 1, characterised in that the sucrose concentration is at least 750 g/l.

3. Process according to Claim 1, characterised in that the procedure is carried out under continuous or semicontinuous operation.

**Patentansprüche**

1. Verfahren zur gleichzeitigen aeroben Herstellung von Gluconsäure, Fruktose und oligosacchariden, bei dem man auf Saccharoselösungen Aspergillus niger-Mikroorganismen einwirken läßt und die Lösung auf einem pH-Wert von etwa 5 hält, und zwar durch regulierte Zugabe einer basischen Flüssigkeit, die die Gluconsäure in Glukonat umwandelt, dadurch gekennzeichnet, daß:
   - in der verdünnten Saccharoselösung durch Fermentation des Aspergillus niger ein Bodensatz hergestellt wird,
   - wenn die Glukosekonzentration etwa 10 g/l beträgt oder wenn die Konzentration an $GF_2$-Triosiden fast ihre Höchstgrenze erreicht hat, die Saccharosekonzentration auf den proliferierenden Aspergillus niger-Zellen auf mehr als 400 g/l erhöht wird,
   - die Mikroorganismen vom Nährboden abgeschieden werden, bevor die Saccharosekonzentration verbraucht ist, um eine Hydrolyse der Oligosaccharide zu verhindern,
   - die Oligosaccharide und die Fruktose von der Gluconsäure oder vom Glukonat getrennt werden.

2. Verfahren nach Anspruch 1, dadurch gekennzeichnet, daß die Saccharosekonzentration mindestens 750 g/l beträgt.

3. Verfahren nach Anspruch 1, dadurch gekennzeichnet, daß im Dauer- bzw. Halbdauerbetrieb gearbeitet wird.